# EUROPEAN PATENT SPECIFICATION

(11) **EP 1 768 579 B1**
(45) Date of publication and mention of the grant of the patent: **14.08.2013**
(21) Application number: 05754581.6
(22) Date of filing: 23.06.2005
(51) Int. Cl.: A61B 17/17

(54) **APPARATUS FOR REPAIRING SEPARATIONS IN THE CAPSULAR LABRUM STRUCTURE**
GERÄT ZUR REPARATUR VON ABLÖSUNGEN DES LABRUMS DER GELENKKAPSEL
DISPOSITIF DE REPARATION DE SEPARATIONS AU NIVEAU DE LA STRUCTURE DU BOURRELET GLENOIDE

(30) Priority: 24.06.2004 US 582087 P
(43) Date of publication of application: 04.04.2007
(73) Proprietor: T.A.G. Medical Devices - Agriculture Cooperative Ltd., 25130 Doar-Na Oshrat (IL)
(72) Inventor: OREN, Ran, 25130 Doar Na Oshrat (IL); LAFOSSE, Laurent, F-74940 Annecy-Le-Vieux (FR)
(74) Representative: Dennemeyer & Associates S.A.
(86) International application number: PCT/IL2005/000673
(87) International publication number: WO 2006/001010

(56) References cited:
- US-A- 4 744 353
- US-A- 5 584 839
- US-A- 5 700 266
- US-A1- 2004 073 227
- US-E- R E36 020

## Description

### FIELD AND BACKGROUND OF THE PRESENT INVENTION

The present invention relates to an apparatus for repairing separations in the capsular labrum structure. The invention is particularly useful in arthroscopic procedures, and is therefore described below with respect to such a procedure,
In the human shoulder, the capsular tendon structure is normally attached to the anterior (front) face of the glenoid neck. When a dislocation of the shoulder joint occurs, the dislocation tears tendons from the inferior (lower) glenoid rim, as well as from the anterior (front) glenoid rim. To restore stability to the shoulder, it is necessary to reattach the tissue as nearly as possible to its original anatomic position.

Soft tissue re-fixation by open surgery is performed from the extra-articular side and applies the tissue to the lateral anterior glenoid, achieving contact over a small but crucial surface. A direct anterior approach through the capsular structure without retracting overlaying tissues to expose the glenoid neck poses a grave hazard of severing vascular-plexus nerve structures.

Arthroscopic techniques practiced at present use only the standard and safe portals, which do not permit access to the glenoid neck. Therefore in arthroscopic procedure, the tissues are brought back to contact only along the rim of the glenoid, without any fixation to the anterior surface of the glenoid neck, leaving a gap between the glenoid rim fixation and the non-involved tissue on the cortical neck. The problem is not apparent from the intra-articular view during arthroscopic reconstruction.

Accessing the glenoid neck for drilling presents problems particularly because the anterior face of the glenoid rim protrudes outwardly of the glenoid neck. A straight drill guide inserted through the standard portal runs almost parallel to the surface of the glenoid neck and therefore cannot normally be used for drilling bores in the glenoid neck during an arthroscopic reconstruction operation. Viewing of the glenoid neck is also problematical as the protruding rim obstructs the field of vision.

Examples of various procedures and apparatus for repairing separations in the capsular labrum structure are described in US-RE-36020, US 2004/0073227, and US 4744353, but these are all subject to one or more of the above drawbacks.

### OBJECTS AND BRIEF SUMMARY OF THE PRESENT INVENTION

According to the present invention, there is provided a device as defined in claim 1 for use in repairing separations in a capsular labrurn structure, in which the head of the humerus bone separates from the glenoid cavity of the glenoid, comprising: a drill guide having a proximal end formed with a handle, and a distal end formed with a surface configuration for engaging the posterior face of the rim of the glenoid to properly fix the position of the drill guide with respect to the posterior face of the glenoid rim; said drill guide being formed with a passageway through said distal end, which passageway is sized and configured for receiving a drill for drilling through the glenoid a bore having an entry point at said posterior face of the rim of the glenoid, and an exit point spaced below said protrusion in the anterior face of the rim of the glenoid.

According to the invention, the surface at the distal end of the drill guide is formed with a recess to receive the posterior face of the rim of the glenoid, and a long nose extending distally from the recess adapted to overlie and engage the posterior face of the rim of the glenoid. In addition, the surface at the distal end of the drill guide is formed with a recess to receive the posterior face of the rim of the glenoid, and a nose to overlie and engage the posterior face of the rim of the glenoid.

According to further features in the described embodiment of such a device, the passageway through the handle and the distal end of the guide is also sized and configured for receiving a suture deployer for deploying a suture through the passageway to the anterior face of the rim of glenoid.

According to a still further aspect of the present invention, there is provided apparatus for use in repairing separations in a capsular labrum structure, in which the head of the humerus bone separates from the glenoid cavity of the glenoid, comprising a drill guide having a proximal end engageable with a drill for guiding its position, and a distal end insertable into a bore formed in an anterior face of a glenoid rim, the drill guide being configured to align a drill at its proximal end with a corresponding anterior face of the glenoid neck, just inwardly of the glenoid rim, when the distal end of the guide is inserted into the bore formed in the anterior face of the glenoid rim.

As will be described more particularly below, the latter devices are particularly useful to drill the holes through the glenoid neck, which is today problematical because of the protruding anterior face of the glenoid rim as mentioned above, thereby permitting the holes through the rim of the glenoid to be drilled according to conventional techniques.

Further features and advantages of the invention will be apparent from the description below.

### BRIEF DESCRIPTION OF THE DRAWINGS

The invention is herein described, by way of example only, with reference to the accompanying drawings, wherein:
Fig. 1 is an anterior (front) view of the capsular labrum structure in the left shoulder, and illustrates in particular the glenoid cavity from which the head of the humerus bone separates in a shoulder dislocation, as well as example of an array of bores to be formed in the glenoid for repairing such a separation;
Fig. 2 is a section view along line II - - II of Fig. 1, to show the curved configuration of the bores formed in the glenoid;
Fig. 3 diagrammatically illustrates one type of drill which may be used for producing the curved bores in the glenoid of Figs. 1 and 2;
Fig. 4 diagrammatically illustrates another type of drill that may be used to drill the curved bores in Figs. 1 and 2;
Fig. 5 is an enlarged fragmentary view illustrating the distal tip of the drill of Fig. 4;
Fig. 6 illustrates another technique that may be used for drilling the continuous bores in Figs. 1 and 2;
Fig. 7 is a view similar to that of Fig. 1 but illustrating another arrangement of bores formed in the glenoid rim and glenoid neck of the glenoid;
Fig. 8 is a section view along VIII -- VIII of Fig. 7;
Fig. 9 illustrates one manner of forming the bores in the glenoid neck in view of the problem caused by the protruding anterior face of the glenoid rim;
Figs. 10-14 illustrate other arrangements that may be used to drill the bores in the anterior face of the glenoid neck in view of the protruding rim problem;
Fig. 15 is a front view of Fig. 14;
Fig. 16 is a view of the capsular labrum structure, similar to that of Figs. 1 and 7, illustrating a plurality of the bores drilled through the glenoid rim, but only one of the bores drilled through glenoid neck by using the apparatus of Fig. 17;
Fig. 17 is a sectional view along line XVII - - XVII of Fig. 16, and illustrating one form of the inventive apparatus for use in drilling the bore in the glenoid neck of Fig. 16;
Fig. 18 illustrates the apparatus of Fig. 17 as also including a suture deployer for deploying a suture via the drill guide through the drilled bore;
and Fig. 19 illustrates the capsular labrum structure as including a plurality of sutures arranged according to the Cassiopeia Pattern produced by use of the apparatus of Figs. 17 and 18.

It is to be understood that the foregoing drawings, and the description below, are provided primarily for purposes of facilitating understanding the conceptual aspects of the invention and possible embodiments thereof, including what is presently considered to be a preferred embodiment. In the interest of clarity and brevity, no attempt is made to provide more details than necessary to enable one skilled in the art, using routine skill and design, to understand and practice the described invention. It is to be further understood that the embodiments described are for purposes of example only, and that the invention is capable of being embodied in other forms and applications than described herein the invention being defined by the appended claims.

### DESCRIPTION OF PREFERRED EMBODIMENTS

The main steps in the surgical procedure disclosed are as follows:
1 - Liberate the soft tissue from the bone;
2 - Abrade the glenoid rim and the glenoid neck to create a bleeding bone;
3 - Drill the bores on the glenoid rim and the glenoid neck;
4 - Deploy the anchors;
5 - Pass the sutures; and
6 - Tighten and tie knots (according to the anchor type).

Steps 1, 2, 4, 5 and 6, are fairly standard operations and may be performed using standard instruments. The present invention concerns mainly step 3, namely drilling bores in the glenoid rim and also in the glenoid neck. Thus, as described earlier, the present invention creates a larger surface of tissue fixation, rather than merely a line of tissue fixation, on the bone in order to provide a large area of healing and to achieve strong tissue-to-bone reattachment in an arthroscopic operation. The disclosuren also provides novel methods and apparatus for drilling the bores in the glenoid neck in an arthroscopic operation despite the problems mentioned above, for accessing and viewing the glenoid neck, because of the protruding anterior face of the glenoid rim.

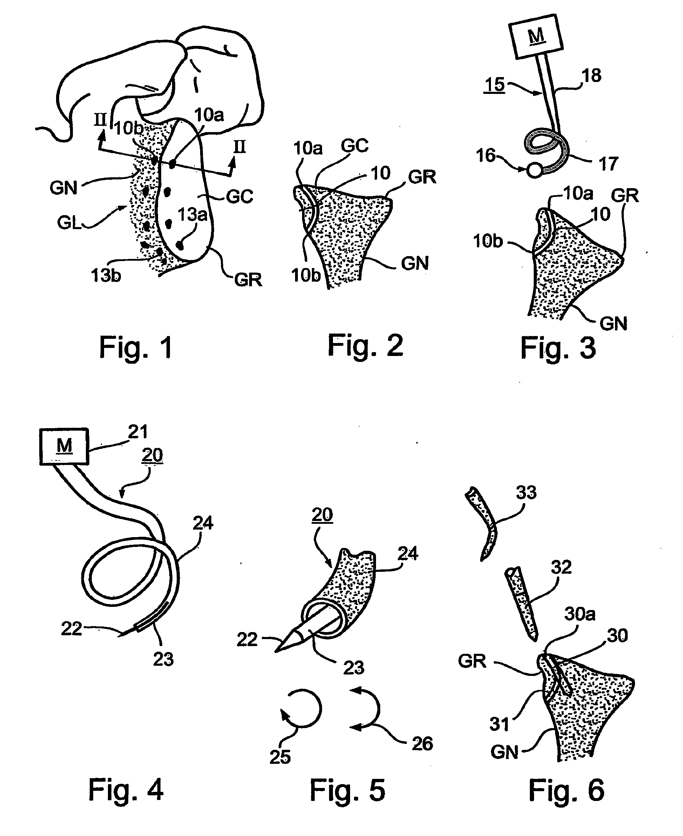

Fig. 1 illustrates one arrangement of bores, more particularly illustrated in the sectional view of Fig. 2, which may be produced in the glenoid neck GN and in the glenoid rim GR to provide the relatively large surface of tissue fixation for achieving strong tissue-to-bone reattachment of the humerous bone with respect to the glenoid cavity GC in an arthroscopic operation. Thus, as shown in Figs. 1 and 2, the anterior face of the glenoid GL is formed with a plurality of curved bores 10 having an entry end at 10a through the glenoid rim GR, and an exit end 10b through the glenoid neck GN. In the example illustrated in Fig. 1, the glenoid GL is formed with four of such curved bores, as shown by entry points 10a-13a in the glenoid rim, and exit points 10b-13b in the glenoid neck, but it will be appreciate that a larger or smaller number of such bores could be produced, according to the particular case.

The entry points 10a-13a of the bores thus define a first plurality of anchor points on the anterior face of the glenoid rim GL for attaching the glenoid GL to the capsular labrum structure; whereas the exit points 10b-13b of the bores define a second plurality of anchor points in the corresponding anterior face of the glenoid neck GN.

Fig. 3 diagrammatically illustrates one type of drilling device, generally designated 15, which may be used for drilling the curved bores shown at 10 in Fig. 2. Thus, drilling device 15 includes a motor M coupled to a drill bit 16 by means of a flexible shaft 17 enclosed within a deformable sleeve 18. An example of such a drill construction is described in US Patent 4,541,423. It will be seen that such a drill is capable of drilling a curved bore through a bone, as more particularly described in the above-cited patent

Figs. 4 and 5 illustrate another drill construction which may be used for this purpose. The drill construction illustrated in Figs. 4 and 5, therein generally designated 20, includes a motor 21 couple to a pointed drilling bit or tip 22 by means of a flexible coupling 23 enclosed within an outer deformable sleeve 24. Drill bit or tip 22 may be rotated continuously in one direction, as indicated by arrow 25, or may be oscillated in opposite directions as indicated by arrow 26. An oscillating drive may be less problematic in some applications and therefore may be preferable. Such a drill may be used for drilling a curved bore as described above, or for breaking through a side of a straight bore, as described below with respect to Fig. 6.

Fig. 6 illustrates an example wherein each of the bores through the glenoid GL is constituted of a straight section 30 in the anterior face of the glenoid rim merging with an angular section 31 leading to and through the corresponding anterior face of the glenoid neck. As further shown in Fig. 6, the straight section 30 may be formed with a conventional straight drill 32, whereas the angular section 31 may be produced by a drill 33 capable of drilling angular bores. In this case, the entry points 30a produced by the straight drill 32 would constitute the plurality of anchor points in the anterior face of the glenoid rim; whereas the exit points 31b in the angular bore sections 31 would constitute the plurality of anchor points in the corresponding anterior face of the glenoid neck.

The disadvantage of continuous tunnels is the weakening of the bone, as can be seen in Fig.2, because the produced bore or tunnel remains close to the surface of the bone. When a non-absorbable suture is used, a hole remains in the bone for a long duration, creating a permanent weak point. Absorbable sutures are not recommended for this specific application as the procedure involves the passing and tightening of the stitch moving through an arched tunnel (the bore) in the bone, thereby causing friction between the bone and the suture. Absorbable sutures are easily damaged by abrasion.

An alternative technique of using two separate holes is therefore advantageous in many cases for these and other reasons.

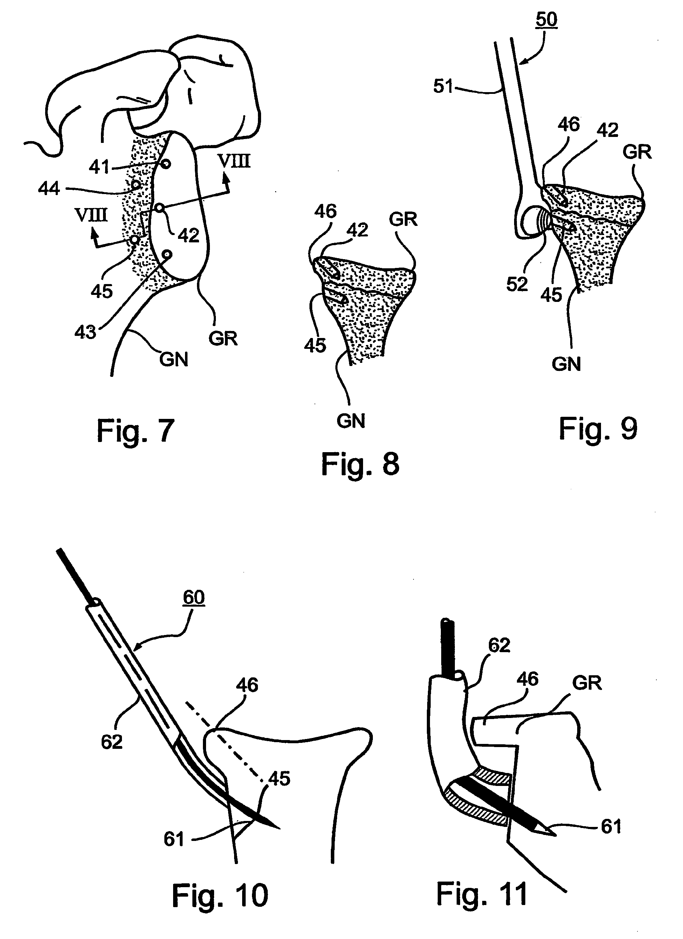

One such alternative technique is shown on Figs. 7 and 8. In this technique, a first group of anchoring points is created by drilling blind bores (e.g., 41-43) in the glenoid rim GR, as with the routine arthroscopic procedure; and the second group of anchoring points is created by drilling, through the corresponding anterior face of the glenoid neck GN another group of blind bores (e.g., 44, 45) in a plane approximately parallel to the bores in the glenoid rim, about 10mm below the rim. With this method, the blind bores are not connected, and there is no need to pass a non-absorbable suture through them. A suitable anchor may be placed in each blind bore, each serving as an independent anchoring point with sutures attached to the anchors for fixing the soft tissue, as described, for example, in US Patent 6,887,259.

Figs. 7 and 8 also demonstrate that substantially less weakening of the bone is involved with this method. Thus, as shown in Fig. 7, bores 44, 45 in the anterior face of the glenoid neck are located medially between bores 41-43 in the corresponding anterior face of the glenoid rim, thereby increasing the amount of bone tissue between two adjacent bores.

Drilling separate bores through the anterior face of the glenoid neck in arthroscopic procedures is problematical because of the protruding anterior face of the glenoid rim which, as described above, makes drill accessibility difficult as well as obstructs the field of view. Figs. 9-19 illustrate various techniques, and also various devices, which may be used to overcome this problem of the protruding anterior face of the glenoid rim, shown on 46 in Fig. 8.

Fig. 9 illustrates a drill, generally designated 50, having a straight shaft 51 for driving a drill bit 52 protruding laterally of shaft 51. Thus, as shown in Fig. 9, the cutter bit 52 may be located to drill the bores 45 in the anterior face of the glenoid neck GN despite the presence of the projecting anterior face 46 of the glenoid rim GR. Drill 50 may thus be used to penetrate the cortex below the glenoid rim 46; if necessary, a curved punch (not shown) may be used to complete the formation the bore 45.

Fig. 10 illustrates a drill, therein designated 60, for drilling the bores (e.g. 45, Fig. 8) in the anterior face of the glenoid neck GN, under the protrusion 46 of the glenoid rim. Drill 60 includes a drill bit 61 enclosed within a curved guide tube 62, in which the distal end of the guide tube is oriented at the desired angle to penetrate the anterior face of the glenoid neck GN under protrusion 46. In the example illustrated in Fig. 10, drill bit 61 is formed with a trocar point. Fig. 11 is an enlarged fragmentary view illustrating the distal tip of drill 60, particularly its drill bit 61 and guide tube 62.

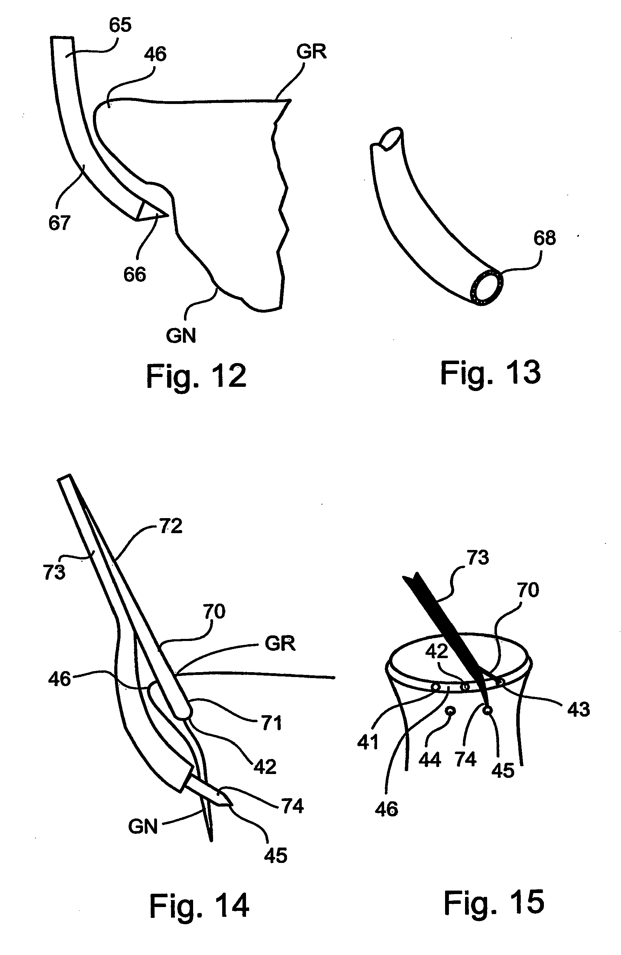

Fig. 12 illustrates the drill, therein designated 65, including a drill tip 66 at the distal end enclosed within a curved guide sleeve 67 for drilling the bores (e.g. 45, Fig. 8) in the anterior face of the glenoid neck GN under the protruding portion 46 of the glenoid rim GR. In this case, drill 66 is preferably of the oscillatory type, rather than the rotating type. Fig. 13 illustrates a similar drill arrangement except the drill is of a hollow construction formed with saw teeth at its distal end, as shown at 68. The drill illustrated in Fig. 13 is also preferably of the oscillatory type.

Fig. 14 illustrates another arrangement for drilling the bores (e.g. 45, Fig. 8) in the anterior face of the glenoid neck GN despite the presence of the protruding portion 46 of the glenoid rim GR which, as indicated earlier, tends to block access of the drill as well as obstruct the view the site of the bores to be formed in the glenoid neck. In this case, the bores 41-43 (Fig. 8) are first drilled in the anterior face of the glenoid rim GR. A guide rod 70 is inserted into one of the bores (e.g. 42) and is used for drilling the corresponding bore (e.g. 45) in the anterior face of the glenoid neck.

Thus, as seen in Fig. 14, guide rod 70 includes a distal end 71 received in the respective bore (e.g. 42) in the glenoid rim GR, and a proximal end 72 which properly locates guide tube 73 such that the drill bit 74 projecting from the distal end of the guide tube engages the anterior face of the glenoid neck at the proper location. This is more clearly shown in Fig. 15, wherein it will be seen that the guide tube 73 for the drill bit 74 is located just under the protruding portion 46 of the glenoid rim GR and medially between two adjacent bores in the corresponding anterior face of the glenoid rim. The arrangement illustrated in Figs. 14 and 15 thus enable the proper formation of the bores in the anterior face of the glenoid neck GN without actually viewing the respective portion of the glenoid neck. It will be appreciated that each of the bores in the glenoid neck is formed in this manner by inserting the distal end of guide rod 70 into the corresponding bore in the glenoid rim GR.

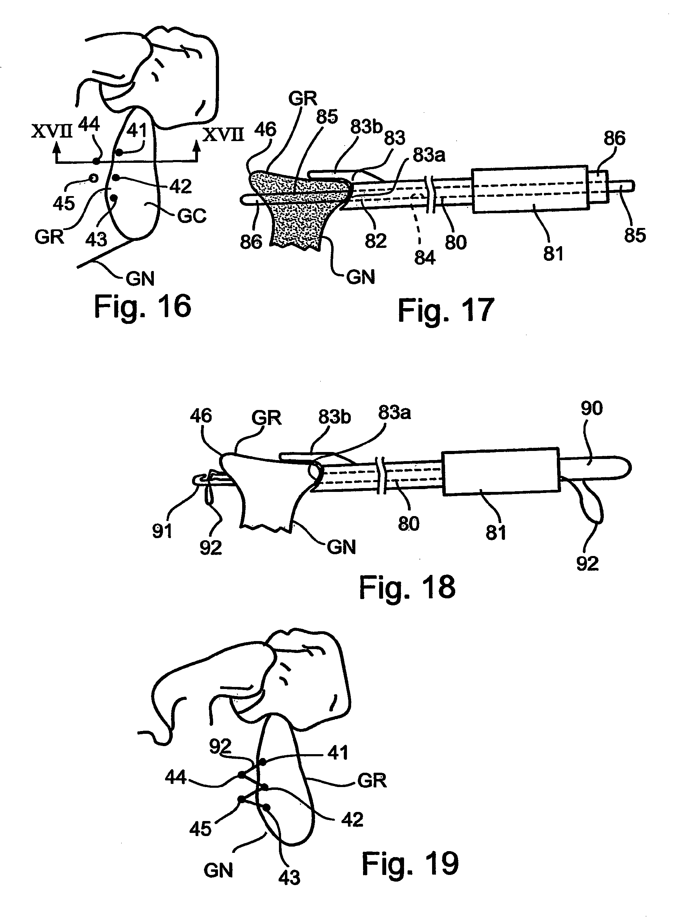

Yet another method for creating such independent anchoring points in the glenoid neck is to access the glenoid neck directly from a posterior, anatomically safe portal, and drill through holes in the bone to reach the desired anterior location. Figs. 16-19 illustrate apparatus which allows the surgeon to target the correct location easily from the posterior portal. Strands of suture introduced nom the posterior side into the holes can be passed to the anterior side and can then be retrieved from that side by suturing instruments to be manipulated as needed to achieve re-attachment of the detached tissue to the bone.

Fig. 16 illustrates a similar layout of bores to be drilled in the glenoid rim GR as in Fig. 7, namely a line of bores 41-43 to be drilled in the anterior face of the glenoid rim GR, and a line of a line of bores 44, 45 to be drilled in the corresponding anterior face of the glenoid neck. The apparatus illustrated in Figs. 17 and 18, as described below, is used for drilling bores 44 and 45 in the anterior face of the glenoid rim via a portal at the posterior face of the glenoid. The section of the glenoid illustrated in Fig. 17 is alone line XVII -- XVII of Fig. 16.

The apparatus according to an embodiment of the invention illustrated in Figs. 17 and 18 includes a drill guide, generally designated 80, having a proximal end formed with a handle 81 for manipulating the guide, and a distal end 82 formed with a surface configuration 83 for engaging a posterior face of the glenoid rim GR to properly fix the position of the drill guide with respect to the glenoid. Drill guide 80 is further formed with a passageway 84 through its handle 81 and distal end 82, which passageway is sized and configured for receiving a long, straight drill 85 to be used for drilling a bore completely through the glenoid neck, entering at its posterior face and exiting through its anterior face.

Thus, as clearly seen in Figs. 17 and 18, the surface configuration 83 at the distal end 82 of guide 80 includes a recess 83a to receive the posterior face of the glenoid rim GR, and a long nose 83b to overlie and engage the posterior face of the glenoid rim. Surface configuration 83 is such that when the distal end 82 of guide 80 engages the posterior face of the glenoid neck, drill tip 86 of drill 85 is precisely located such that the drill tip exits from the anterior face of glenoid neck at a distance of about 5-15mm, preferably at about 10mm, from the outer surface of the anterior face of the glenoid rim GR.

Each of the bores 44, 45 in the anterior face of the glenoid neck GN is formed by the use of drill guide 80 via a posterior portal as described above. The bores 41-43 formed in the corresponding anterior face of the glenoid rim GR may be formed by using conventional straight drills, as described above, either before or after bores 44, 45 are formed in the corresponding anterior face of the glenoid neck GN.

Preferably, drill 85 includes a stop 86 abuttable against the proximal face of handle 81 to limit the further movement of the drill after exiting from the bore formed in the anterior face of the glenoid neck GN.

After guide 80 is used for forming the bores 44, 45 in the anterior face of the glenoid neck GN, as described above, the same guide may be used for deploying sutures through the so-formed bores. Thus, passageway 84 formed in guide 80 is also sized and configured so as to receive a suture deployer, generally designated 90 in Fig. 18, instead of drill 85. Suture deployer 90 includes an eyelet 91 at its distal end for delivering a strand of suture 92 via the posterior portal used for drilling the glenoid neck bores (44, 45) to the anterior side of the glenoid neck bores. The so-delivered sutures may be retrieved from the standard anterior-lateral portal with known devices and used for re-attaching the detached tissue to the bone according to known procedures. Fig. 19 illustrates the Cassiopeia Pattern of sutures 92 produced according to this procedure.

While the invention has been described to several preferred embodiments, it will be appreciated that these are set forth merely for purposes of example, and that many other variations, modifications and applications of the invention may be made within the scoop of the appended claims.

## Claims

1. Apparatus for use in repairing separations in a capsular labrum structure, in which the head of the humerus bone separates from the glenoid cavity of the glenoid (GN), the glenoid (GN) (including a rim (GR) having a posterior face, and an anterior face formed with a protrusion (46) which makes drilling accessibility difficult from the anterior face of the glenoid, said apparatus comprising:
a drill guide (80) having a proximal end formed with a handle (81), and a distal (82) end formed with a surface configuration (83) for engaging the posterior face of the rim (46) of the glenoid to properly fix the position of the drill guide with respect to the posterior face of the glenoid rim (GR);
said drill guide (80) being formed with a passageway (84) through said distal end (82), which passageway is sized and configured for receiving a drill (85) for drilling through the glenoid a bore having an entry point at said posterior face of the rim of the glenoid, and an exit point spaced below said protrusion in the anterior face of the rim of the glenoid;
wherein said surface (83) at said distal end of the drill guide (80) is formed with a recess (83a) to receive said posterior face of the rim (GR) of the glenoid, and a long nose (83b) extending distally from the recess (83a) adapted to overlie and engage said posterior face of the rim of the glenoid
and wherein said surface configuration (83) at the distal end of the guide (80) is adapted to engage said posterior face of the rim (GR) of the glenoid to locate the drill tip, when the drill is received fully inside said passageway (84), at a distance of about 5-15 mm from the outer surface of said protrusion (46) in the anterior face of the rim (GR) of the glenoid.

2. The apparatus according to Claim 1, wherein said distance is approximately 10 mm.

3. The apparatus according to Claim 1, wherein said apparatus further comprises a drill (85) having a shaft dimensioned to pass through said passageway (84) and to exit therefrom at said anterior face of the neck (GN) of the glenoid.

4. The apparatus according to Claim 3, wherein said drill (85) further includes a stop (86) abuttable against the handle (81) to fix the exit point of the drill at the anterior face of the rim of the glenoid.

5. The apparatus according to Claim 1, wherein said passageway (84) through said handle (81) and said distal end of the guide (80) is also sized and configured for receiving a suture deployer (90) for deploying a suture through said passageway to the bore in the anterior face of the rim of the glenoid.

6. The apparatus according to Claim 5, wherein said apparatus further comprises a suture deployer (90) including an eyelet (91) at its distal end for delivering a suture (92) through the anterior face of the rim of the glenoid.

## Patentansprüche

1. Vorrichtung zur Verwendung bei der Reparatur von Trennungen in einer Kapsel-Labrum-Struktur, wobei sich der Kopf des Humerus von der Cavitas glenoidalis des Glenoids (GN) trennt, wobei das Glenoid (GN) einen Rand (GR) mit einer Vorderfläche und einer Rückfläche enthält, der mit einem Vorsprung (46) ausgebildet ist, der eine Bohrzugänglichkeit von der Vorderfläche des Glenoids schwierig gestaltet, wobei die Vorrichtung umfasst:
eine Bohrführung (80) mit einem proximalen Ende, das mit einem Griff (81) ausgebildet ist, und mit einem distalen Ende (82), das mit einer Oberflächenkonfiguration (83) für einen Angriff an der Rückfläche des Randes (46) des Glenoids ausgebildet ist, um die Position der Bohrführung in Bezug auf die Rückfläche des Glenoidrandes (GR) richtig zu fixieren;
wobei die Bohrführung (80) mit einem Durchgang (84) durch das distale Ende (82) ausgebildet ist, wobei der Durchgang von einer solchen Größe und Konfiguration ist, dass er einen Bohrer (85) aufnimmt, um eine Bohrung mit einem Eintrittspunkt an der Rückfläche des Randes des Glenoids und einem Austrittspunkt, der unterhalb des Vorsprungs in der Vorderfläche des Randes des Glenoids mit Abstand angeordnet ist, durch das Glenoid zu bohren;
wobei die Oberfläche (83) an dem distalen Ende der Bohrführung (80) mit einer Vertiefung (83a), um die Rückfläche des Randes (GR) des Glenoids aufzunehmen, und mit einer langen Nase (83b) ausgebildet ist, die sich distal von der Vertiefung (83a) erstreckt und so ausgelegt ist, dass sie die Rückfläche des Randes des Glenoids überlagen und an dieser angreift,
und wobei die Oberflächenkonfiguration (83) am distalen Ende der Führung (80) so ausgelegt ist, dass sie an der Rückfläche des Randes (GR) des Glenoids angreift, um die Bohrerspitze zu positionieren, wenn der Bohrer zur Gänze innerhalb des Durchgangs (84) in einer Distanz von ungefähr 5 bis 15 mm von der Außenfläche des Vorsprungs (46) in der Vorderfläche des Randes (GR) des Glenoids aufgenommen ist.

2. Vorrichtung nach Anspruch 1, wobei die Distanz ungefähr 10 mm beträgt.

3. Vorrichtung nach Anspruch 1, wobei die Vorrichtung ferner einen Bohrer (85) mit einer Welle enthält, die so dimensioniert ist, dass sie den Durchgang (84) passiert und aus diesem an der Vorderfläche des Halses (GN) des Glenoids austritt.

4. Vorrichtung nach Anspruch 3, wobei der Bohrer (85) darüber hinaus einen Anschlag (86) enthält, der am Griff (81) anlegbar ist, um den Austrittspunkt des Bohrers an der Vorderfläche des Randes des Glenoids zu fixieren.

5. Vorrichtung nach Anspruch 1, wobei der Durchgang (84) durch den Griff (81) und das distale Ende der Führung (80) auch von einer solchen Größe und Konfiguration ist, dass er eine Fadenbereitstellungseinheit (90) zum Bereitstellen eines Fadens durch den Durchgang zur Bohrung in der Vorderfläche des Randes des Glenoids aufnimmt.

6. Vorrichtung nach Anspruch 5, wobei die Vorrichtung ferner eine Fadenbereitstellungseinheit (90) umfasst, die eine Öse (91) an ihrem distalen Ende zum Zuführen eines Fadens (92) durch die Vorderfläche des Randes des Glenoids enthält.

## Revendications

1. Appareil destiné à être utilisé pour réparer des séparations dans une structure de bourrelet capsulaire, dans lesquelles la tête de l'humérus se sépare de la cavité glénoïde de la glène (GN), la glène (GN) comprenant un rebord (GR) ayant une face postérieure, et une face antérieure comportant une protrusion (46) qui rend l'accessibilité d'un forage difficile depuis la face antérieure de la glène, ledit appareil comprenant :
un guide-mèche (80) ayant une extrémité proximale dotée d'une poignée (81), et une extrémité distale (82) présentant une configuration de surface (83) conçue pour s'emboîter avec la face postérieure du rebord (46) de la glène afin de correctement fixer la position du guide-mèche par rapport à la face postérieure du rebord de la glène (GR) ;
ledit guide-mèche (80) étant doté d'un passage (84) à travers ladite extrémité distale (82), lequel passage est dimensionné et configuré pour recevoir une mèche (85) afin de forer un trou dans la glène ayant un point d'entrée au niveau de ladite face postérieure du rebord de la glène, et un point de sortie espacé en dessous de ladite protrusion dans la face antérieure du rebord de la glène ;
où ladite surface (83) au niveau de ladite extrémité distale du guide-mèche (80) est dotée d'un renfoncement (83a) pour recevoir ladite face postérieure du rebord (GR) de la glène, et d'une longue extrémité avant (83b) s'étendant en direction distale par rapport au renfoncement (83a) qui est adaptée pour recouvrir et s'emboîter avec ladite face postérieure du rebord de la glène
et où ladite configuration de surface (83) au niveau de l'extrémité distale du guide (80) est adaptée pour s'emboîter avec ladite face postérieure du rebord (GR) de la glène afin de localiser la pointe de la mèche, où la mèche est entièrement reçue à l'intérieur dudit passage (84), à une distance d'environ 5-15 mm par rapport à la surface externe de ladite protrusion (46) dans la face antérieure du rebord (GR) de la glène.

2. Appareil selon la revendication 1, où ladite distance est d'approximativement 10 mm.

3. Appareil selon la revendication 1, où ledit appareil comprend en outre une mèche (85) ayant une tige qui est dimensionnée pour passer à travers ledit passage (84) et pour ressortir de celui-ci au niveau de ladite face antérieure du col (GN) de la glène.

4. Appareil selon la revendication 3, dans lequel ladite mèche (85) comprend en outre une butée (86) pouvant jouxter la poignée (81) afin de fixer le point de sortie de la mèche au niveau de la face antérieure du rebord de la glène.

5. Appareil selon la revendication 1, dans lequel ledit passage (84) à travers ladite poignée (81) et ladite extrémité distale du guide (80) est également dimensionné et configuré pour recevoir un système de déploiement de suture (90) afin de déployer une suture à travers ledit passage jusqu'au trou dans la face antérieure du rebord de la glène.

6. Appareil selon la revendication 5, où ledit appareil comprend en outre un système de déploiement de suture (90) comprenant un oeillet (91) au niveau de son extrémité distale afin de délivrer une suture (92) à travers la face antérieure du rebord de la glène.
